## Europäisches Patentamt

## European Patent Office

## Office européen des brevets

(19)

(11) Numéro de publication: **0 086 731**
**A2**

(12)

# DEMANDE DE BREVET EUROPEEN

(21) Numéro de dépôt: **83440014.5**

(22) Date de dépôt: **15.02.83**

(51) Int. Cl.³: **A 61 M 5/14**

(30) Priorité: **16.02.82 ES 509640**

(43) Date de publication de la demande: **24.08.83**
**Bulletin 83/34**

(84) Etats contractants désignés: **AT BE CH DE FR GB IT LI LU NL SE**

(71) Demandeur: **Bozal Gonzalez, José Luis, Isaac Peral 30, Majadahonda Madrid (ES)**

(72) Inventeur: **Bozal Gonzalez, José Luis, Isaac Peral 30, Majadahonda Madrid (ES)**

(74) Mandataire: **Poupon, Michel, Cabinet Arbousse Bastide 20, rue de Copenhague, F-67000 Strasbourg (FR)**

(54) **Système de pompage volumétrique pour infusion dosée au moyen d'une membrane élastique, pour applications médicales.**

(57) Système de pompage volumétrique pour infusion dosée au moyen d'une membrane élastique pour des applications médicales avec dosage automatique et contrôlé des liquides utilisés pour infusions veineuses et/ou artérielles, qui comprend un corps de pompe à l'intérieur duquel est placé le corps principal d'un élément jetable comportant une membrane en caoutchouc naturel ou latex à grande capacité de distension, caractérisé en ce que, essentiellement, le groupe de pompage comporte une plaque de membrane (14) et une porte (12) avec sa fermeture et des moyens d'alarme, lesquels retiennent le corps principal de l'élément jetable, dont la membrane est capable de distension par pression hydrostatique vers l'extérieur selon une mesure déterminée par un logement prévu dans la plaque de membrane de la pompe et en ce que le fond de ce logement a à sa base un dispositif de soufflet où l'air est comprimé pour pousser le liquide logé à l'intérieur de la membrane élastique vers la partie jetable, tout en contrôlant les opérations de remplissage et de vidange avec des éléments de soupape placés à l'entrée et à la sortie, commandés par la pompe, de telle sorte que la chambre se remplisse par pression hydrostatique, lorsque l'entrée est ouverte et la sortie fermée, la sortie se fermant jusqu'à atteindre une pression suffisante, puis s'ouvrant pour permettre que le liquide coule vers le patient à la pression requise.

1

SYSTEME DE POMPAGE VOLUMETRIQUE POUR INFUSION DOSEE AU
MOYEN D'UNE MEMBRANE ELASTIQUE, POUR APPLICATIONS MEDI-
CALES.

La présente invention a pour objet un système
de pompage volumétrique pour infusion dosée au moyen
d'une membrane élastique pour des applications médicales avec dosage automatique et contrôlé des liquides utilisés pour infusions veineuses et/ou artérielles, qui
comprend un corps de pompe à l'intérieur duquel est placé le corps principal d'un élément jetable comportant
une membrane en caoutchouc naturel ou latex à grande
capacité de distension.

Les moyens utilisés à présent en médecine prévoient le rétablissement de l'équilibre des constantes
vitales de l'individu au moyen de l'infusion de liquides
depuis l'extérieur vers une voie intraveineuse, intra-
artérielle ou intra-péritonéale, d'une manière précise
et contrôlée. L'administration par infusion est appliquée à de nombreux liquides différents, de constitutions
très diverses et pour de multiples applications prévues
dans la pratique médicale. La valeur du rythme du fluide
à infuser à un patient est déterminée en fonction de ses
caractéristiques vitales et selon l'évolution de sa maladie.

Un des systèmes existants pour infuser des

2

liquides aux patients consiste dans l'utilisation d'un piston. Ce type de système prévoit un récipient intermédiaire, dans la ligne bouteille-patient, avec deux soupapes à l'entrée et à la sortie de celui-ci. A l'intérieur agit un piston à vitesse réglable et pouvant être contrôlé électroniquement, ledit cylindre ou piston réalisant des charges successives par succion du fluide et des décharges par impulsion du liquide contenu entre les soupapes d'entrée et de sortie.

Le rythme d'infusion du fluide dépend dans ce système de la vitesse de déplacement du piston à l'intérieur de sa chemise, contrôlée électroniquement. Quoique la précision de ce système puisse être considérée comme acceptable, il entraine un énorme risque d'embolie gazeuse provoquée par l'infusion d'air dans le système sanguin, au cas où le piston aspire de l'air au lieu de liquide. En général ces systèmes incluent une alarme qui arrête automatiquement son fonctionnement, l'alarme fonctionnant lorsqu'elle détecte des gouttes. L'arlarme peut faillir tout simplement par embuement ou par fixation de gouttes contre les murs de la chambre de dégouttement et alors l'on injecterait de l'air au patient. De plus, l'élément jetable, constitué par un ensemble de tuyaux, soupapes, piston, chemise du piston, etc..., constituant toute la ligne du fluide depuis la bouteille jusqu'au patient supposait des coûts très élevés.

Un autre dispositif, parmi les divers instruments de ce type est la pompe péristaltique, agissant dans un tuyau flexible allant de la bouteille jusqu'au patient. La pompe agit seulement sur l'extérieur du tuyau et par conséquent il n'y a pas de risque substantiel d'introduire d'éléments étrangers dans le liquide intraveineux. Les machines à pompes péristaltiques agissent en comprimant le tuyau flexible de la solution dans un certain nombre de points et poussant les les points de compression en direction du patient. Dans cette zone de compression le tuyau doit avoir une configuration particulière pour permettre le déplacement du volume du fluide vers le patient avec une précision acceptable. Après une période de fonctionnement de la pompe, le tuyau n'a plus une flexibilité et une résistance absolues, ce qui provoque des imprécisions dans le volume de liquide infusé. Le risque d'embolie gazeuse déjà cité subsiste, car le liquide entre dans la conduite par succion.

On s'est alors acheminé vers une vessie extensible, c'est-à-dire un appareil capable d'emmagasiner et plus tard de pousser par ses propres moyens une quantité prédéterminée de liquide. Ces désavantages en sont :

- un volume à infuser prédéterminé, car il n'a qu'un seul point d'entrée et de sortie, sans possibilité de circulation continuelle à l'intérieur ;

- imprécision dans le pompage, parce que l'impulsion vient de la vessie elle-même lorsqu'elle tend à reprendre sa géométrie initiale : cette impulsion est variable selon le degré de gonflement de la vessie.

Une autre alternative de pompe péristaltique est un appareil conçu par R.SCOTT TURNER. Il prévoit une combinaison d'un élément jetable ou cassette et d'un contrôleur pour maintenir le niveau de fluide de la solution intraveineuse toujours au-dessous d'une valeur déterminée au préalable. L'appareil de Turner ne prévoit pas la possibilité d'une force poussant le fluide vers l'intérieur du patient ; il est supposé que cette fonction se réalise par gravité ; donc on prévoit que le niveau de fluide dépasse le niveau considéré pour qu'il n'y ait pas de risque pour le patient ; l'élément jetable de l'appareil de Turner fait partie du passage du fluide vers le patient et comporte une chambre de mesure avec un volume maximum pré-établi. Cette cassette comprend deux sections en plastique rigide avec une section élastique en polyuréthane intermédiaire disposée entre elles, le fluide passant à travers la cassette qui compacte le matériel plastique sur l'une des sections en plastique rigide.

Le plastique flexible se plie toujours entre deux configurations ; dans une d'elles la chambre n'a pas essentiellement de volume, dans l'autre, la chambre subit une expansion jusqu'au volume pré-établi. La cassette est pourvue aussi d'une entrée et d'une sortie vers la chambre, la fonction essentielle du contrôleur étant celle d'agir au moyen des soupapes, sur la quantité de fluide passant à travers la cassette. En particulier, le contrôleur ouvre l'entrée, permet le remplissage de la chambre de mesure et après ferme l'entrée ; ensuite la soupape de fermeture s'ouvre pour permettre l'écoulement du liquide de la chambre de mesure vers le patient ; enfin, la fermeture se bloque et le processus recommence.

Le contrôleur a, pour les soupapes d'entrée et de sortie, des tiges séparées dont le bout est aiguisé. Pour fermer la soupape, le contrôleur déplace la tige à travers un trou du convercle en plastique rigide.

L'appareil de Turner offre des caractéristiques limitant sérieusement son application et donc sa réalisation à un niveau industriel.

Les trous en polyuréthane faisant partie des soupapes peuvent souffrir une modification permanente de leur configuration en raison de l'attaque constante des tiges de la soupape du contrôleur. Après le retrait des tiges à l'intérieur du contrôleur ouvrant la soupape il se peut que le trou ne reprenne pas,obligatoirement, son profil primitif, ce qui supposerait que la soupape resterait fermée.

Par ailleurs, cette forme de distorsion constante peut conduire à ce que le polyuréthane élastique faisant partie de la chambre de mesure lâche en revenant à la configuration prévue de cette chambre, ce qui supposerait un changement du volume contenu dans la cassette.

Aussi, la configuration aiguisée des bouts des tiges du contrôleur, lesquelles attaquent constamment la membrane en plastique rigide, provoque très facilement des coupures sur celle-ci, ce qui empêche le fonctionnement de l'appareil.

5

Un essai pour trouver une solution aux problèmes posés par l'appareil de Turner est l'engin conçu par BAXTER-TRAVENOL, qui a recours à une cassette utilisant un élément élastique et un contrôleur d'infusion intraveineuse. Par rapport à l'appareil de Turner essentiellement il constitue une amélioration de l'élément jetable et du contrôleur.

L'élément jetable est toujours fait en deux sections en plastique rigide entre lesquelles il y a une membrane élastique, dans ce cas ci à base de caoutchouc naturel ou latex, tandis que dans l'appareil de Turner elle était en plastique, et avec la pièce supérieure comportant des orifices sur lesquels agissent les tiges des électro-aimants contrôlant l'entrée et la sortie ; ces tiges agissent sur ladite membrane.

Malgré des sévères caractéristiques de fonctionnement, l'appareil présente divers inconvénients limitant sa vialibité en cas d'utilisation continuelle. Les tiges des électro-aimants continuent à attaquer la membrane élastique, bien que dans ce cas elles ne soient pas aiguisées et elles continuent à présenter les inconvénients précités, à savoir déformation de l'orifice et usure de la membrane.

Par ailleurs, le caractère volumétrique de ce système devient très limité en raison de deux aspects : le premier et le plus important est que le corps en plastique rigide configurant la partie antérieure présente un enfoncement qui devrait être toujours rempli de liquide à infuser, ce qui est incertain car la membrane élastique lors de sa compression a tendance à dépasser la position plate. En outre, dans la ligne de sortie du fluide sur la zone de la soupape, constituée par la membrane, au fur et à mesure que le dépôt élastique se vide, il y a un moment où le latex provoque un effet de succion sur la ligne citée.

La membrane élastique en latex se distend jusqu'à venir en contact avec l'élément en plastique qui unit cette membrane à un autre élément en plastique. Les deux éléments en plastique sont unis entre eux par soudure ultrason. Selon la qualité de la soudure entre les deux éléments en plastique il peut y avoir jeu ou une cavité, ce qui fera que le volume défini par la membrane élastique lors de sa distension sur ledit élément en plastique ne soit pas parfaitement défini, d'où imprécision de la dose. Par exemple, si le jeu est seulement de 0.1 mm, le volume de la membrane de 1 $cm^3$ et le diamètre utile de la membrane de 50mm, le volume additionnel une fois rempli serait de 0,2 $cm^3$, ce qui suppose un volume réel de 1,2 $cm^3$, c'est-à-dire une erreur de 20 %.

Un autre aspect important qu'il faut considérer dans les deux appareils précités est l'absence d'une garantie absolue de non-injection d'air au patient, ce qui comporte le risque d'une embolie gazeuse. Par ailleurs, la force d'impulsion du fluide au patient est essentiellement la gravité, donc ils restent limités à des infusions intraveineuses, car les infusions artérielles nécessitent une plus grande pression, car il n'y a aucun élément de pompage ou d'impulsion du liquide contenu à l'intérieur de l'élément jetable.

De même que TURNER et BAXTER-TRAVENOL, ANDROS dans son appareil, part d'un dispositif comportant des moyens de conduite des liquides pouvu d'autres moyens à l'extrémité réalisant les fonctions des soupapes d'entrée et de sortie du liquide. Cependant et afin de pouvoir pomper avec ses propres moyens, sans avoir besoin de la pression hydrostatique, on a changé la membrane élastique par une autre semi-rigide pouvant être mécaniquement déformée par l'action d'une tige commandée de manière cyclique par une came actionnée avec un moteur électrique. Dans le cycle de pompage ladite tige presse la membrane semi-rigide en pompant du liquide vers l'extérieur. Lors du mouvement de recul de la tige, la membrane semi-rigide reprend sa géométrie initiale en provoquant une dépression dans son intérieur qui aspire le liquide provenant de l'entrée. En d'autres termes, le remplissage de l'élément conducteur du liquide ne se fait pas seulement par pression hydrostatique mais il y a aussi une aspiration de celui-ci réalisée par l'ensemble de pompage. Cela suppose que ledit équipement doit comporter un élément d'alarme de bouteille vide, pour empêcher l'aspiration d'air et par conséquent son pompage vers le patient.

L'idée d'utiliser un fluide à pression pour pomper un autre fluide, les deux fluides étant séparés par une membrane élastique, fut déjà utilisée par Litton dans son costume spatial.

Selon l'invention, on remédie à tous ces inconvénients de l'art antérieur en proposant un système de pompage du type décrit ci-dessus, caractérisé en ce que, essentiellement, le groupe de pompage comporte une plaque de membrane et une porte avec sa fermeture et des moyens d'alarme, lesquels retiennent le corps principal de l'élément jetable, dont la membrane est capable de distension par pression hydrostatique vers l'extérieur selon une mesure déterminée par un logement prévu dans la plaque de membrane de la pompe et en ce que le fond de ce logement a à sa base un dispositif de soufflet où l'air est comprimé pour pousser le liquide logé à l'intérieur de la membrane élastique vers la partie jetable, tout en contrôlant les opérations de remplissage et de vidange avec des éléments soupape placés à l'entrée et à la sortie, commandés par la pompe, de telle sorte que la chambre se remplisse par pression hydrostatique, lorsque l'entrée est ouverte et la sortie fermée, la sortie se fermant jusqu'à atteindre une pression suffisante, puis s'ouvrant pour permettre que le liquide coule vers le patient à la pression requise.

La différence fondamentale entre les pompes BAXTER-TRAVENOL et celle de l'invention consiste dans la cassette : la chambre de mesure est placée dans une des deux moitiés en plastique rigide, ce qui n'est pas le cas de la cassette de l'invention, de sorte que dans cette dernière la chambre de mesure se trouve dans la pompe d'infusion.

Cette différence entre les deux dispositions suppose les avantages fonctionnels et de fabrication suivants :

8

1. La tolérance entre les deux pièces en plastique soudées par ultrason, se traduit par une grande imprécision dans le dosage dans le cas de BAXTER-TRAVENOL, tandis que cet effet ne se produit pas dans la pompe de l'invention.

2. Etant donné que la chambre de mesure est placée dans la pompe d'infusion, dans ce cas ci l'on peut disposer d'un système de tarage du volume de cette chambre, ce qui corrige les éventuels défauts de manufacture de ladite chambre.

3. En fonction des points 1 et 2 ci-dessus, la présente réalisation permet de plus grandes tolérances dans la partie mécanique (moules pour couler les pièces en plastique de la cassette et aussi de la chambre de mesure de la pompe) ce qui réduit le coût de fabrication.

Par rapport aux dispositifs ANDROS et LITTON, les différences fondamentales sont :

1. La pression du fluide pompeur est celle de l'intérieur du récipient contenant ledit fluide, dans ces dispositifs, tandis que dans le cas de la présente pompe, cette pression résulte de la compression de l'air atmosphérique lors de chaque cycle de pompage.

2. Chez LITTON, la disposition de la membrane et des éléments de soupape fait que la pression du fluide de pompage se règle elle-même en fonction du débit du fluide pompé.

Dans la pompe de l'invention il n'y a aucun mécanisme d'auto-réglage ; les cycles de pompage et de charge sont réglés électroniquement depuis l'extérieur.

Il est évident que n'importe quel dispositif d'infusion doit comporter des éléments soupape nécessaires pour régler l'entrée et la sortie du liquide.

Conformément aux principes de fonctionnement de la présente invention, ce système comporte une partie structurelle de pompage proprement dit et un élément jetable échangé pour chaque infusion. L'élément jetable comprend une partie

9

rigide et une planche circulaire de latex, à grande capacité de distension, solidement réunies au moyen de conduites convenablement placées. Le liquide entre dans la zone intermédiaire entre la pièce en plastique et la planche en latex provoquant ainsi le remplissage et le gonflement de l'espace compris entre elles, dilatant ladite planche jusqu'à atteindre un volume pré-établi par une configuration taillée avec une grande précision dans le corps de la pompe d'infusion proprement dite ; ce volume fournit le caractère volumétrique et une grande précision au système, empêchant l'imprécision constatée dans la cassette de TURNER.

D'autre part la pompe prévoit deux tiges convenablement situées à l'entrée et à la sortie du liquide, contrôlées électroniquement depuis un tableau de commande pour l'usager. L'effet de pompage de ce liquide vers la sortie est obtenu par un électro-aimant, contrôlée aussi électroniquement, agissant sur une chambre fermée ou soufflet lequel exerce une pression sur la membrane ou latex en forçant la sortie du liquide contenu vers la conduite d'injection de liquide vers le patient, la soupape de sortie étant ouverte, ce qui permet la sortie de tout le liquide contenu dans le dépôt élastique ou son fractionnement en parties plus réduites en fonction du rythme d'infusion choisi depuis le panneau de commande. Cet électro-aimant est nécessaire lorsqu'il faut une pression de sortie assez élevée.

L'élément jetable est constitué par un corps principal configurant des conduites d'entrée et de sortie vers des cavités où l'on place des bouchons déformables sur lesquels agiront les tiges des électro-aimants. La conduite d'entrée en amont permet la sortie vers la partie antérieure, vers une membrane distensible supportée par un couvercle supérieur, lequel a dans la surface centrale, couverte par la membrane citée, un grand nombre d'orifices vers un canal arrière lequel reste défini entre ce corps central et un couvercle arrière, ce qui configure aussi les conduites d'entrée et de sorti depuis le logement des bouchons déformables jusqu'à la configuration centrale précitée.

Les limites de distension de la membrane sont limitées par la surface perforée du corps central et, lors de la distension, par la cavité définie à l'intérieur du groupe de pompage. Le contrôle du passage de fluide, d'entrée et de sortie, sont opérés par deux bouchons en matériau synthétique déformable bouchant les conduites formées par ledit corps central.

En outre, la configuration non-symétrique de l'élément jetable configure une forme unique pour introduire la partie jetable dans la pompe en raison de la non symétrie des guides de positionnement incorporés.

Pour procéder au contrôle nécessaire de la cassette, de même que dans les appareils précédents, l'on procède à divers mouvements d'ouverture et de fermeture des soupapes d'entrée et de sortie. En fermant la soupape de sortie et en ouvrant celle d'entrée la pression hydrostatique du fluide provoque la distension de la membrane élastomère à l'intérieur de la chambre de mesure installée dans le groupe de pompage. Une fois remplie de liquide la membrane élastique de la partie jetable et les soupapes d'entrée et de sortie du liquide fermées, on commande l'électro-aimant du dispositif soufflet, jusqu'à ce que la pression à l'intérieur de celui-ci atteigne la valeur correspondante au point d'équilibre entre la force de l'électro-aimant et la pression de l'air ; alors la soupape de sortie s'ouvre et le liquide commence à sortir de la partie jetable à une pression supérieure à la pression atmosphérique. Le groupe de pompage est commandé par un sélecteur de rythme, lequel, si le fluide à infuser est au-dessous d'un débit pré-établi réalise des ouvertures et fermetures successives de la soupape de sortie permettant que tout le liquide contenu dans la chambre sorte, lequel se distribue en différentes injections, ce qui rend possible une plus grande régularité de l'administration de celui-ci. Si le débit de l'in-

11

fusion est d'un certain volume l'injection se produit dans sa totalité avec une seule ouverture de la soupape de sortie. Une fois vidé le contenu du liquide dans la chambre d'expansion le groupe de pompage ferme la soupape de sortie et ouvre la soupape d'entrée pour réaliser un nouveau remplissage de la chambre ; ces opérations ont lieu d'une manière continuelle et consécutive.

La pompe comprend des éléments de détection de la fin du contenu de la bouteille, de fermeture à l'entrée et à la sortie de la partie jetable, de mauvaise fermeture de la porte, de batterie vide et de mauvais fonctionnement électronique, en provoquant l'arrêt de la pompe et des signaux lumineux et accoustiques d'alarme.

On comprendra mieux l'invention à l'aide de la description ci-après d'un mode de mise en oeuvre de l'invention, en référence aux dessins annexés dans lesquels :

- la figure 1 représente une vue générale du groupe de pompage proprement dit,

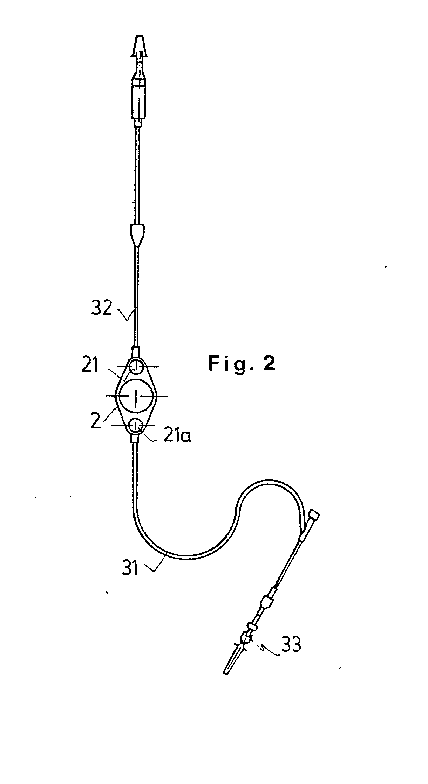

- la figure 2 représente l'ensemble de la partie jetable, avec les autres éléments accessoires,

- la figure 3 représente une disposition pratique du système de pompage préconisé,

- la figure 4 représente une vue verticale, le centre du groupe de pompage, y compris le groupe jetable dans son intérieur et en position de fonctionnement,

- la figure 5 représente une vue horizontale dudit groupe dans la position de la figure précédente,

- la figure 6 représente une vue en hauteur et en coupe du corps central de la partie jetable.

Le dispositif de l'invention, consiste en un système de pompage composé essentiellement par deux groupes, la pompe proprement dite (1) et l'élément jetable (2), ce dernier élément configurant le seul contact avec le liquide à infuser au patient.

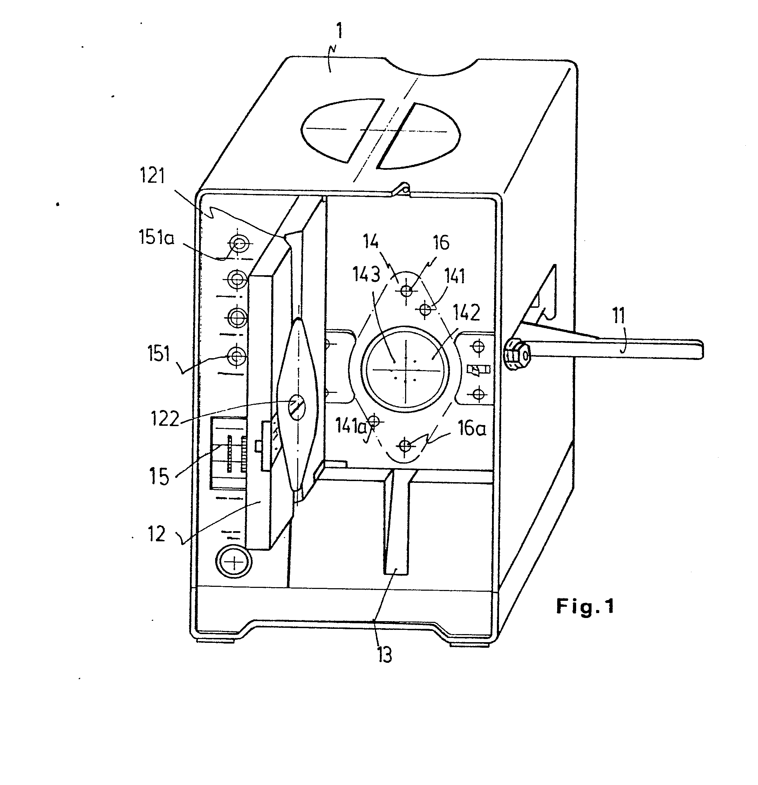

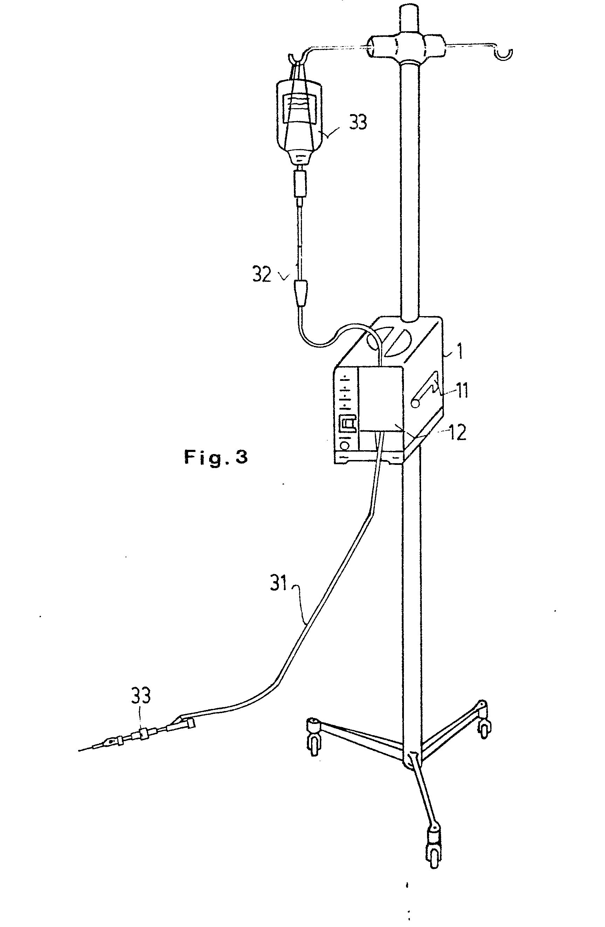

En se fondant sur les figures 1 et 3 on constate que la pompe présente une carcasse parallélépipède (1) dont la partie antérieure s'ouvre sur une porte (12) comportant dans sa partie intérieure une cavité (14) d'une forme correspondant à l'élément jetable (2), celui-ci pouvant se disposer dans ladite cavité au moyen de deux plots de guidage (141) et (141a) et rester étroitement uni à celle-ci par la fermeture de la porte (12) de la pompe citée. Par les deux extrémités de cet élément jetable sortent deux conduites (32) et (31) allant respectivement vers la bouteille de remplissage de liquide (33) et vers la conduite d'injection de liquide au patient (31), terminé par l'aiguille hypodermique (33).

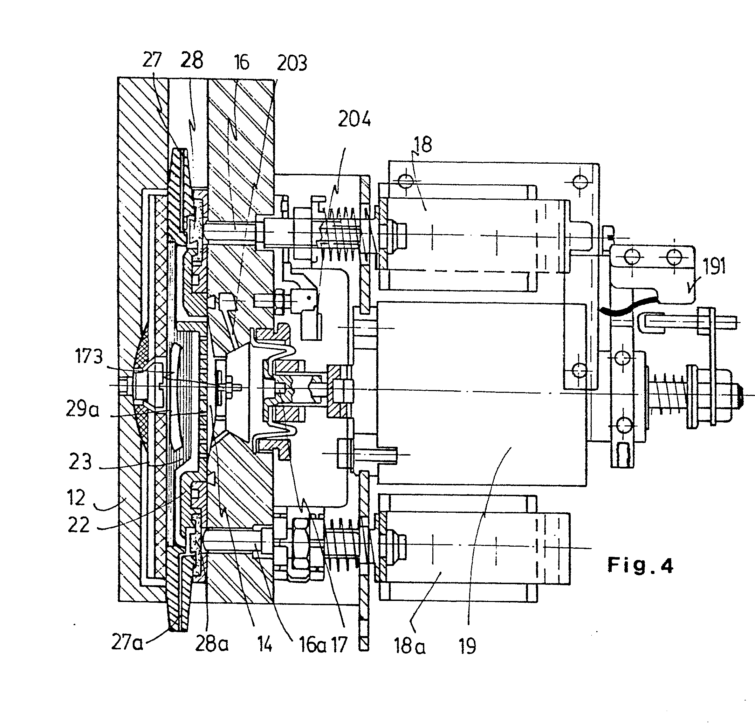

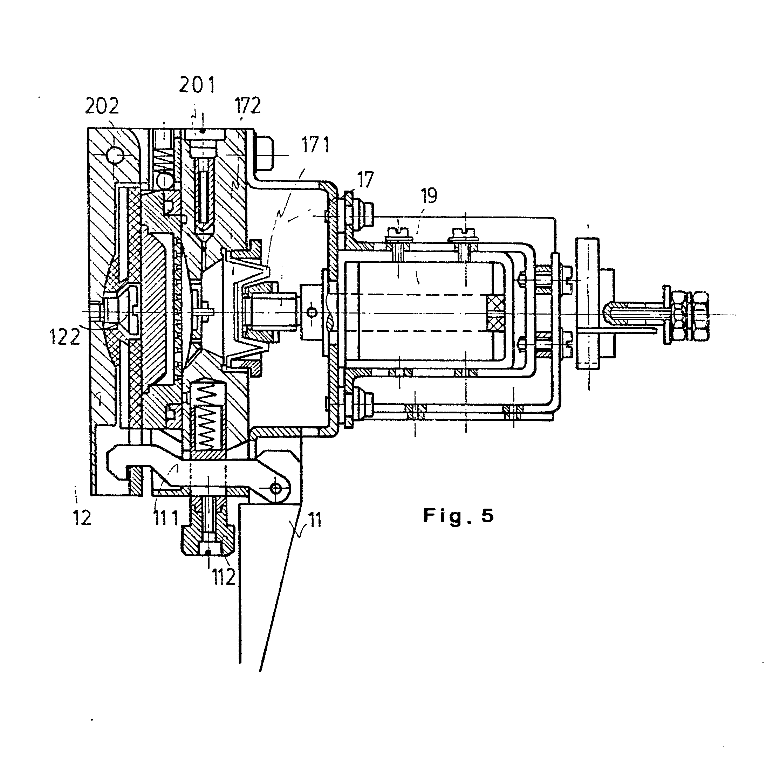

La configuration générale de la pompe d'infusion est telle qu'elle figure représentée aux figures 1,4 et 5, et suppose un corps où a lieu la réception du liquide à infuser et son pompage selon la dose indiquée au préalable par le sélecteur de rythme (15), avec à l'extérieur des indicateurs de commande et d'alarme (151). Ce corps principal de la pompe est constitué essentiellement par deux éléments fonctionnellement différents. Le premier est le groupe de pompage comportant la plaque de membrane (14) à l'intérieur de laquelle est logé le corps principal de la partie jetable (2) et le groupe agissant qui comporte trois électro-aimants (18), (18a) et (19) chargés de contrôler l'entrée et la sortie du liquide et exerçant une surpression sur celui-ci respectivement.

Le groupe de pompage est constitué à son tour par la plaque de membrane (14), la porte de fermeture (12), les deux tiges des soupapes de passage (16) et (16a) et un interrupteur d'alarme de porte ouverte. Dans la plaque de membrane il y a un logement (142) de contour en forme de calotte sphérique de capacité déterminée et qui remplit des fonctions de chambre d'expansion de la membrane élastique (25) de la partie jetable, dont la chambre d'expansion reste pleine de liquide lorsque la soupape d'entrée est ouverte, du fait de la pression hydrostatique de celui-ci. Dans la base de cette plaque il y

13

a une cavité tronconique (172) qui est la base d'un soufflet (171) où se comprime un volume d'air destiné à donner de l'impulsion au liquide logé à l'intérieur de la membrane (25) lorsque la soupape de sortie sera ouverte.

La base de ladite plaque comporte deux éléments de positionnement (14) et (14a) susceptibles d'être inclus dans les orifices (26) et (26a) de la partie jetable (2).

Vers les deux extrémités, supérieure et inférieure, il y a des sorties (13) pour les conduites correspondantes du circuit hydraulique ; aussi, la porte (12) présente en vis à vis des entailles correspondantes (121), en correspondance avec celles déjà citées (13), pour serrer les conduites d'entrée et de sortie (32) et (31) respectivement.

La fermeture de ladite porte est assurée par un double dispositif de sécurité représenté par les références (111) et (112), dont le dernier bout est représenté par le levier (11) ; ledit dispositif comporte une alarme qui manifeste l'état d'ouverture de ladite porte. Sur sa face intérieure il comporte la chambre d'une rotule (122) pour stabiliser la pression exercée sur la partie jetable.

Le groupe agissant est constitué par deux électro-aimants (18) et (18a) qui contrôlent les soupapes d'entrée et de sortie du liquide (21) et (21a) respectivement, par l'action exercée par les tiges (16) et (16a) de chacun des électro-aimants sur les soupapes ou bouchons élastiques cités se trouvant dans l'élément rejettable (2). Ce groupe comporte aussi un électro-aimant d'une plus grande puissance lequel commande la tige (17) qui actionne le soufflet (171), déjà cité, et un interrupteur de fin de course (191) dont le signal dûment utilisé par la plaque de contrôle électronique détecte une obstruction dans la ligne d'entrée du liquide, la fin de la bouteille ou une obstruction dans la sortie du liquide vers le patient.

Les applications d'infusion intra-artérielle supposent une surpression supérieure à la sortie de l'infusion, ce qui rend nécessaire que le système de pompage soit capable de fournir le liquide nécessaire aux pressions atteintes par le corps humain ; le procédé utilisé par le soufflet (171) est basé sur l'augmentation de la pression d'un fluide gazeux à température légèrement constante lorsque le volume du récipient qu'il contient diminue ; dans ce cas-ci ledit fluide est l'air atmosphérique.

L'énergie nécessaire pour produire la pression s'obtient au moyen de l'électro-aimant (19), lequel développe un travail utilisé pour déplacer la surface mobile faisant partie d'un récipient fermé de contour tronconique contenant l'air que l'on veut compresser ; ce récipient fermé, de capacité variable, a été désigné jusqu'à présent "soufflet", avec la référence (171) et la chambre intérieure (172). La communication entre ledit soufflet et la chambre d'expansion de la partie jetable (2) se fait au moyen de quelques petits orifices (143) qu'elle possède ; de cette manière, la pression atteinte à l'intérieur du soufflet sera la même que celle du liquide à pomper à l'intérieur de la partie jetable.

Il faut noter l'utilisation d'une petite plaque mobile (173) constituant la base de la chambre d'expansion de la membrane, dont le déplacement est parfaitement réglable, capable d'absorber les différences de volume de la chambre, dûes aux tolérances de fabrication de la chambre de mesure. Avec un réglage préalable, un débit d'infusion pré-établi et étant donné que dans cette pompe le volume d'expansion de la membrane se trouve dans l'appareil lui-même et pas dans la partie jetable, il est possible d'absorber les erreurs provenant du procédé de mécanisation de la chambre d'expansion ou de mesure.

Comme indiqué précédemment le contrôle de ce système est fait par une mémoire électronique, dans laquelle est imprimée la séquence à suivre par les électro-aimants pour son actionnement, conformément au débit signalé dans le sélecteur de rythme ; cette séquence est décrite ci-après.

15

Tout d'abord la soupape d'entrée s'ouvre (28) les autres restant en repos le temps nécessaire pour la distension complète de la membrane élastique (25) par effet de la pression hydrostatique du fluide. Une fois remplie de liquide la membrane élastique jetable et les soupapes d'entrée (28) et de sortie (28a) du liquide, fermées, on actionne l'électro-aimant (19) du soufflet (172) jusqu'à ce que la pression à l'intérieur de celui-ci atteint la valeur correspondante au point d'équilibre entre la force de l'électro-aimant (19) et la pression de l'air ; alors on ouvre la soupape de sortie (28a) et le liquide commence à sortir de la partie jetable (2) à une pression supérieure à la pression atmosphérique ; le volume balayé par le soufflet (172) est pré-établi par la relation qu'il a avec le volume occupé par la chambre d'expansion de la membrane, donc, au cas où celle-ci ne comporterait pas de liquide, le positionnement initial de la tige (17) de l'électro-aimant permet de le détecter au moyen d'un micro-interrupteur (191).

Le fonctionnement ci-dessus décrit varie légèrement si le rythme indiqué par le sélecteur a une valeur inférieure à une valeur pré-établie ; dans ce cas là, la soupape de sortie au lieu de s'ouvrir et de permettre que la totalité du liquide contenu dans la chambre sorte, produit une succession d'ouvertures et de fermetures, ceci distribuant le liquide à flots, ce qui permet une plus grande régularité dans son administration.

Ce système de pompage est complété moyennant l'introduction de deux soupapes d'air ce qui assure que la pression dans la chambre d'expansion de la membrane élastique de la partie jetable ne soit pas différente de la pression atmosphérique à certains moments.

Lorsque la soupape de passage du liquide à la partie jetable (soupape d'entrée) est ouverte, la membrane commence à se distendre au fur et à mesure que le liquide entre et par conséquent cette diminution du volume provoque une augmentation de la pression à l'intérieur du soufflet, avec la possibilité d'empêcher que la membrane de la partie jetable ne se dilate jusqu'à heurter le contour de la calotte (14). Pour résoudre ce problème, on a prévu une soupape de passage de l'air (204) commandée par la tige (16) de l'électro-aimant d'entrée de liquide. L'intérieur du soufflet reste en communication avec l'atmosphère au moyen de la conduite (203).

En outre, pendant la course de recul du piston du soufflet il peut se produire l'effet contraire, c'est-à-dire que en augmentant le volume géométrique du soufflet la pression à l'intérieur de sa chambre diminuerait ; bien que cette aspiration ne pourrait jamais avoir un effet sur la ligne de sortie du liquide, elle pourrait avoir des conséquences sur la membrane élastique de la partie jetable, laquelle pourrait être aspirée. Cet effet est évité en plaçant une autre soupape de passage de l'air (201) permettant le passage de celui-ci vers l'extérieur.

On a décrit jusqu'à présent en détail le groupe de pompage de ce système ; on abordera maintenant le groupe agissant de celui-ci, chargé d'agir physiquement sur le corps principal de la partie jetable en réalisant l'ouverture et la fermeture des soupapes d'entrée et de sortie de celui-ci et aussi d'agir sur le soufflet de pression avant décrit. Par ailleurs il comporte un dispositif pour détecter par exemple n'importe quelle obstruction dans la ligne d'entrée et de sortie du liquide et la fin de la bouteille.

Les électro-aimants agissant sur les soupapes d'entrée, de sortie et le soufflet sont trois, placés de sorte que s'il y a une faille dans le système d'alimentation ou dans la plaque de contrôle électronique, la situation de repos de ceux-ci empêche l'entrée et la sortie du liquide et aussi la compression du soufflet.

A un moment donné s'il n'y a pas de liquide dans la bouteille, ou si la conduite d'entrée de la partie jetable est obstruée, l'alarme d'obstruction de la ligne d'entrée ou de fin de bouteille se déclenche, laquelle détecte ce moment là, car si le liquide n'est pas entré dans la membrane jetable et si celle-ci ne s'est pas distendue, l'axe de l'électro-aimant du soufflet parcourera un espace plus grand, car la capacité de la membrane n'est pas occupée, et ce déplacement plus grand sera détecté par un micro-interrupteur (191) placé près de l'axe de l'électro-aimant du soufflet, commandé par la tige de celui-ci. Le signal émis par ledit micro-interrupteur est utilisé par la plaque de contrôle électronique pour éviter l'ouverture de la soupape de sortie et allumer une alarme (15) placée dans le panneau antérieur du groupe de pompage (1) et bloquer le fonctionnement de la pompe.

Par ailleurs, lors de la dernière phase de liquide, lorsque la soupape de sortie se ferme, et que l'électro-aimant du soufflet est encore commandé, le micro-interrupteur doit avoir dû être actionné, car il ne doit pas rester de liquide dans la membrane. Si dans ces circonstances, le micro-interrupteur n'a pas été actionné cela suppose que la membrane est encore remplie de liquide, donc qu'il y a occlusion à la sortie. Le signal dudit micro-interrupteur est utilisé par le contrôle électronique pour arrêter le fonctionnement de la pompe, et en même temps il émet une alarme accoustique et lumineuse.

Le contrôle électronique de commande des électro-aimants et la source d'alimentation de ceux-ci sont organisés à partir d'une mémoire qui enregistre l'information concernant les divers rythmes d'infusion ; la direction de cette mémoire se fait dans un générateur de directions en accord avec un générateur de rythme et selon le rythme d'infusion, choisi avec un clavier (15). La synchronisation de ces circuits se réalise à l'aide d'une horloge mère qui produit un signal selon une fréquence spécifique. La sortie de la mémoire peut être bloquée par le circuit d'alarme s'il y a lieu et elle attaque directement le circuit d'activation des électro-aimants ; les alarmes, par exemple, sont visualisées à l'extérieur dans les indicateurs lumineux (151) et accoustiquement avec un son intermittent.

L'alimentation de cette pompe prévoit la possibilité de la brancher directement au réseau, indépendamment de l'incorporation intérieure d'une série de batteries lui permettant une autonomie absolue, dans les cas de déplacements urgents, etc...

Jusqu'à présent on a décrit le fonctionnement et les caractéristiques de la pompe d'infusion proprement dite. Maintenant on décrira l'élément jetable (2) accouplé à celle-ci. Cet élément jetable, comme dit précédemment, comporte un corps principal dont l'entrée (32) communique avec la bouteille de liquide (33) et la sortie avec la conduite (31) transportant le liquide vers l'aiguille hypodermique (33) injectée au patient.

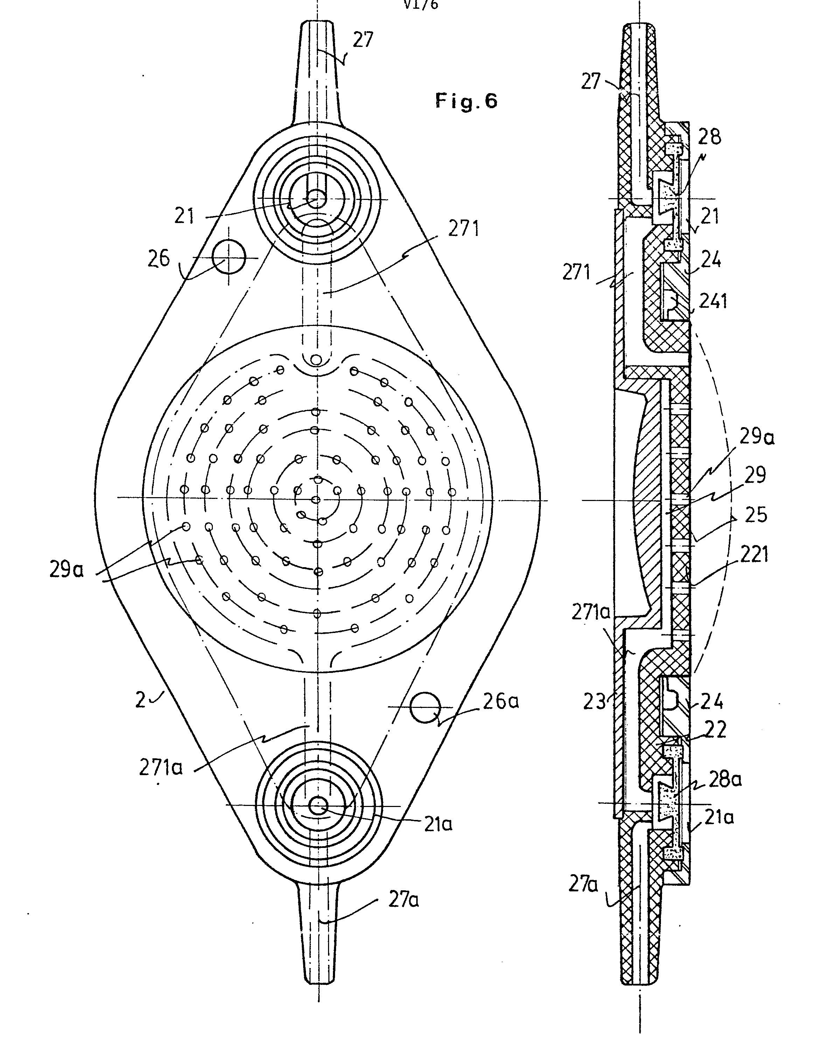

Comme on peut le voir sur la figure 6, le corps principal de la partie jetable est constitué par un corps central (23) où se trouve une membrane en latex (25) à grande capacité de distension, fixée au moyen du couvercle supérieur (24) audit corps central. Le canal d'entrée du liquide (27) configuré par le corps central lui-même (22), de section cylindrique, a un diamètre calibré pour permettre l'entrée du volume de liquide nécessaire pour la dilatation de la soupape d'entrée. L'entrée se fait essentiellement par gravité et par conséquent la dilatation de la membrane est due à la pression hydrostatique du liquide en amont. Donc, s'il n'y a pas de liquide, par exemple en fin de la bouteille à infuser, l'air ne peut pas dilater la membrane car elle reste plate et aplatie sur la partie plate (221) du corps principal (22) ce qui donne un volume nul entre cette partie plate et elle-même. Etant donné que la membrane ne se dilate pas, l'action de l'électro-aimant de surpression n'a aucun effet de pompage, donc la planche reste collée à la surface et le pompage d'air est impossible. Lors de la réalisation et du fonctionnement de la pompe lorsque cela aura lieu le dispositif de détection de fin de bouteille décrit précédemment se mettra en marche, ce qui bloque le fonctionnement de la pompe afin d'éviter un travail inutile, en visualisant l'état d'alarme de fin de bouteille pour prévenir le patient.

Comme représenté à la figure 6, le canal d'entrée (27) communique directement vers une cavité où est logé le bouchon (28), il parcourt une certaine distance (271) pour sortir dans l'espace compris entre la zone plate (221) du corps central (22) et la membrane extensible (25). Cette surface plate (221) présente un grand nombre de trous (29a). Le corps central (22) est pourvu dans la partie arrière d'un couvercle (23) en combinaison avec lequel il forme les canaux (271) et (271a) et le canal (29). Ce canal (29) qui se prolonge à la sortie dans le canal (271a) et celui-ci, avec le bouchon (28a), interposé au préalable, vers la conduite de sortie (27a), termine en pénétrant dans le corps principal. Il n'y a pas d'inter-communication entre le canal (29) et le canal d'entrée (271). Cette zone d'orifices permet le passage du liquide et constitue la limite pour que la course de la planche en repos soit nulle. Cette planche (25) reste en position au moyen du couvercle antérieur (24), lequel supporte aussi les bouchons (28) et (28a) entre la planche et la coupe principale (22), et comporte des orifices (21) et (21a) où agissent les tiges (16) et (16a) des électro-aimants (18) et (18a).

Les bouchons (28) et (28a) ont une tige agissant sur la conduite à fermer avec une configuration tronconique, dont la base plus grande agit sur la conduite citée. Le bouchon d'entrée (28) agit sur le canal (27) d'entrée. Par ailleurs, le bouchon de sortie (28a) agit sur le canal (271a). De cette manière et au moment où s'établit la pression de pompage sur la partie jetable, celle-ci se traduit sur le bouchon (28a) par une force qui agit sur la zone en pente vers la conduite (271) ; la composante de force agissant sur le bouchon peut se décomposer alors en deux : une composante horizontale qui reste annulée par une autre opposée de la même valeur, et une perpendiculaire à l'orifice, favorisant sa fermeture. Lorsque la partie jetable est vide et que dans la conduite de sortie (27a) il y a une pression légèrement supérieure, en raison de la pression sanguine du patient, le bouchon (28a) produit le même effet que la précédente, ladite pression agissant selon une force perpendiculaire à la conduite (271a), sur laquelle s'adaptera le bouchon (28a), ce qui favorise sa fermeture.

Aussi, il faut souligner que le couvercle supérieur (24) a un canal circulaire (241) dans la zone proche de l'orifice central où reste retenue la membrane (25) ; le but de la conduite (241) est d'agir en tant qu'épanchement des désajustements éventuels lors de l'usinage, ce qui permet que les excédents de membrane restent inclus dans ledit canal, car cette membrane (25) ne doit pas dépasser les limites de ce canal afin d'obtenir une soudure parfaite au delà de ses extrémités.

Pour ce qui est des orifices de passage (29a) faisant communiquer la conduite antérieure (29) avec l'espace compris entre le corps principal (22) et la membrane (25), il faut remarquer que leur section est légèrement tronconique; leur base majeure correspond à la zone face à la membrane. Cette conicité facilite une pression plus réduite, lors du pompage, dans la zone correspondante vers la conduite (29), ce qui dans la zone intérieure de la membrane empêche la possibilité de petites bulles d'air par effet de cavité, ce qui pourrait avoir lieu au cas où le liquide pourrait se dilater dans une certaine mesure.

En dernier lieu, il faut signaler que les unions des couvercles supérieur et inférieur au corps central se font par soudure ultrason sans avoir recours, par conséquent, à du matériel d'apport extérieur, ce qui pourrait dégrader la qualité médicale des matériaux. En outre, le type de matériel utilisé dans toute la ligne de conduite de l'élément jetable respecte les recommandations les plus récentes concernant la pose des scellés médicaux formulées dans différents pays.

Pour terminer on signalera certaines caractéristiques techniques spécifiques qui montrent l'intérêt particulier de ce système de pompage comme par exemple la sécurité intrinsèque, la gamme l'utilisation et la précision, qui sont une conséquence directe du système ; d'autres, comme la régularité sont le fruit des diverses améliorations et des innovations réalisées tout le long du développement de l'invention; les résultats finaux ont été soigneusement mesurés en laboratoire d'essais.

La précision de ce système est de 1% du volume préétabli dans les conditions de pression à la sortie les plus défavorables, ceci étant déterminé par les déphasages ou déséquilibres minimums pouvant se produire lors du gonflement de la chambre d'expansion, tout en considérant que celle-ci est limitée par le creux de la pompe. Si on suppose des conditions normales de fonctionnement, ce pourcentage peut atteindre 0,1% et en fait dépend de la précision de l'usinage du contre-moule.

Il offre une haute régularité car le temps mort de remplissage de la partie jetable est très réduit. Pour produire ce remplissage, avec une vitesse suffisante, il faut considérer que la chambre se remplit par pression hydrostatique, c'est-à-dire par le poids de la colonne de liquide, et pour cela il faut que la bouteille soit au-dessus de la pompe, à une hauteur de 40cm, selon les essais réalisés.

La gamme d'application dépend du sélecteur de rythme et permet d'infuser par exemple, depuis 1 $cm^3$/heure, jusqu'à 1000 $cm^3$/heure, sans oublier que si le rythme est trop bas, le contenu de la chambre peut être fractionné en diverses infusions, afin de mieux répartir l'administration du liquide au patient.

Pour l'autonomie, cette caractéristique dépend bien entendu du rythme choisi ; la pompe offre un système de charge de batteries lorsqu'elle est branchée au réseau.

Comme il a été déjà décrit, le système peut avoir une alarme d'obstruction de la ligne d'entrée ou de fin de bouteille, de même qu'une alarme de porte ouverte et d'obstruction de la sortie, le tout complété par une alarme montrant la situation de la batterie et le mauvais fonctionnement électrique. Il a été signalé en outre qu'il n'est pas nécessaire d'avoir une alarme d'embolie gazeuse, car le système étant extrêmement sûr, il est impossible d'injecter de l'air au patient.

23

REVENDICATIONS

1. Système de pompage volumétrique pour infusion dosée au moyen d'une membrane élastique pour des applications médicales avec dosage automatique et contrôlé des liquides utilisés pour infusions veineuses et/ou artérielles, qui comprend un corps de pompe à l'intérieur duquel est placé le corps principal d'un élément jetable comportant une membrane en caoutchouc naturel ou latex à grande capacité de distension, caractérisé en ce que, essentiellement, le groupe de pompage comporte une plaque de membrane (14) et une porte (12) avec sa fermeture et des moyens d'alarme, lesquels retiennent le corps principal de l'élément jetable, dont la membrane est capable de distension par pression hydrostatique vers l'extérieur selon une mesure déterminée par un logement prévu dans la plaque de membrane de la pompe et en ce que le fond de ce logement a à sa base un dispositif de soufflet (171) où l'air est comprimé pour pousser le liquide logé à l'intérieur de la membrane élastique vers la partie jetable, tout en contrôlant les opérations de remplissage et de vidange avec des éléments de soupape placés à l'entrée et à la sortie, commandés par la pompe, de telle sorte que la chambre se remplisse par pression hydrostatique, lorsque l'entrée est ouverte et la sortie fermée, la sortie se fermant jusqu'à atteindre une pression suffisante, puis s'ouvrant pour permettre que le liquide coule vers le patient à la pression requise.

2. Système de pompage selon la revendication 1, caractérisé en ce que l'énergie nécessaire pour le pompage en comprimant la membrane (25) de la partie jetable s'obtient à partir d'un électro-aimant qui développe un travail utilisé pour déplacer une surface mobile ou soufflet faisant partie du logement fermé contenant l'air à comprimer, ce logement communiquant avec la chambre d'expansion (142) de la membrane jetable (25) moyennant de petits orifices (143), la pression développée par ce soufflet étant suffisante pour le pompage

24

de liquides depuis l'intérieur de la partie jetable vers le patient sous n'importe quelle condition de pression artérielle ou veineuse, les électro-aimants étant réglés par une plaque de contrôle électronique.

3.      Système de pompage, selon les revendications 1 et 2, caractérisé en ce que le fond de la chambre de mesure comporte une petite plaque réglable (173) permettant l'absorption des différences de volume de la chambre dûes au processus d'usinage de celle-ci.

4.      Système de pompage, selon les revendications 1 et 2, caractérisé en ce que le logement fermé par le soufflet comprend une soupape auxiliaire (204) de passage de l'air commandée par la tige (16) de l'électro-aimant d'entrée de liquide, laquelle accélère le processus de remplissage de la partie jetable, en assurant la communication du logement de la membrane avec l'atmosphère, en vue d'obtenir un équilibre de la pression atmosphérique entre le logement de la membrane et la chambre du soufflet et l'atmosphère, pendant le processus de charge de la partie jetable.

5.      Système de pompage, selon les revendications 1,2 et 3, caractérisé en ce qu'il comporte une soupape auxiliaire (201) de passage de l'air, uni-directionnelle, faisant communiquer la chambre du soufflet directement avec l'atmosphère, ceci permettant le passage de l'air vers l'intérieur, en empêchant de la sorte que la pression ambiante soit supérieure à la pression à l'intérieur de la chambre du soufflet et que celui-ci provoque une succion sur la membrane de la partie jetable dans sa course en arrière.

6.      Système de pompage volumétrique, selon les revendications 1 et 2, caractérisé en ce qu'il comporte un dispositif de détection de fin de bouteille constitué par un micro-interrupteur (191) et une plaque de contrôle électronique arrêtant le fonctionnement de la machine lorsque la membrane flexible ne fait pas de distension, l'électro-aimant commandant alors ce micro-interrupteur en raison de la course supérieure de la tige.

7.      Système de pompage volumétrique selon les revendications 1,2 et 6, caractérisé en ce qu'il comprend un dispositif de détection d'une obstruction à la sortie, constitué par le micro-interrupteur (191) et par la plaque de contrôle électronique, lequel émet un signal si le soufflet est commandé et ne se déplace pas suivant un parcours pré-établi, ce qui indique que la chambre d'expansion est pleine, en raison d'une obstruction à la sortie, ledit signal étant utilisé pour émettre des alarmes accoustiques et/ou lumineuses.

8.      Système de pompage selon la revendication 1, dans lequel le contrôle électronique de celui-ci prévoit une mémoire contrôlée depuis un générateur de rythme, susceptible de provoquer l'arrêt selon les alarmes prévues, caractérisé en ce que cette mémoire, pour une valeur basse de rythme prévoit que la soupape de sorite réalise des ouvertures successives, espacées, pour provoquer une distribution du liquide contenu dans la chambre en plusieures infusions, afin d'obtenir une plus grande régularité de l'infusion, le reste des phases développées par le système restant inchangées.

9.      Système de pompage, selon la revendication 1, caractérisé en ce que le corps principal de la partie jetable a un corps central avec des conduites d'entrée (27) et de sortie (27a) du liquide, définissant deux orifices où se logent des bouchons déformables (28,28a) faisant partie des soupapes, sur lesquelles agissent les électro-aimants d'entrée et de sortie, accouplés respectivement à la bouteille d'alimentation et au tuyau d'injection au patient, la conduite d'entrée, en amont, communiquant avec un seul orifice avec le corps central vers la membrane supportée, de même que les bouchons cités, au moyen d'un couvercle supérieur, et ledit corps comportant une zone plate centrale pourvue de plusieurs orifices communiquant avec une conduite arrière fermée par un couvercle inférieur, et avec une sortie vers la soupape située à l'autre bout, de telle manière que le poids du liquide, en ouvrant l'entrée, bombe la membrane en aval, si la sortie a été fermée et le remplissage de liquide s'effectuant en inversant l'ouverture et la fermeture des soupapes, il s'exerce une pression sur la membrane, le liquide sortant poussé vers la sortie.

10. Système de pompage selon la revendication 9, caractérisé en ce que la partie jetable comporte près de ses éléments soupape, et placés en vis à vis, des orifices capables de s'adapter à des guides (26,26a) disposés dans la pompe pour permettre un positionnement précis de la partie jetable, dont l'asymétrie évite la connexion inversée de celle-ci.

11. Système de pompage volumétrique selon la revendication 9, caractérisé en ce que les orifices d'entrée et de sortie de la partie jetable restent définis dans leurs parcours depuis l'origine jusqu'aux éléments soupape au moyen d'une conduite, raccord tronconique formé par le corps principal de la partie jetable elle-même.

12. Système de pompage volumétrique selon la revendication 9, caractérisé en ce que les éléments de soupape ou bouchons ont une configuration tronconique dont la base plus grande est tournée vers l'orifice à boucher.

13. Système de pompage volumétrique selon les revendications 9 et 12, caractérisé en ce que le bouchon d'entrée(28) agit sur la conduite définie par le raccord d'entrée, pour que lors du pompage, avec une plus grande pression dans la conduite en aval, celle-ci exerce une force dont la composante perpendiculaire facilite l'ajustement dudit bouchon à l'orifice.

14. Système de pompage volumétrique selon la revendication 9, caractérisé en ce que les orifices d'entrée et de sortie de la partie jetable dans son parcours depuis les éléments de soupape jusqu'à la configuration centrale, restent définis par les conduites ouvertes dans le corps central, ceux-ci se fermant au moyen d'un couvercle, en rapprochant la conduite d'entrée à la membrane qui se bombe de manière indéfinie, si elle n'est pas comprise dans la pompe, tandis que la conduite de sortie se termine dans une cavité définie sur toute la zone plate centrale par le corps principal de la partie jetable et le couvercle arrière de celui-ci.

15. Système de pompage selon les revendications 9 et 14, caractérisé en ce que les orifices du corps principal dans la zone plate, où est supportée la membrane, présentent une configuration tronconique, dont la base plus grande est orientée vers la face de la membrane et la plus réduite vers la conduite de sortie, afin d'éviter des effets de cavitation.

16. Système de pompage volumétrique selon les revendications 9 et 14, caractérisé en ce que le bouchon de sortie (28a) agit sur la conduite ouverte du corps principal, de manière que lorsque la chambre d'expansion sera vide, et si dans la conduite de sortie il y a une plus grande pression qu'à l'intérieur de celle-ci, la pression se décompose en une force dont la composante perpendiculaire favorise le rapprochement dudit bouchon vers la conduite.

17. Système de pompage volumétrique selon la revendication 9, caractérisé en ce que le couvercle supérieur se présente sous la forme d'un élément pouvu d'une grande ouverture centrale à travers laquelle doit émerger la membrane lors de la dilatation et en ce qu'il réalise, avec le corps principal, des cavités pour retenir les bouchons faisant partie des soupapes, offrant deux orifices correspondants au centre des bouchons cités, à travers lesquels agissent les tiges des électro-aimants d'entrée et de sortie.

18. Système de pompage volumétrique, selon les revendications 9 et 17, caractérisé en ce que le couvercle supérieur définit un entaillage autour de toute la périphérie proche à son point de fixation de la membrane distensible, cet entaillage permettant un jeu des éventuels désajustements de ladite membrane, laquelle en aucun cas ne reste retenue dans la zone qui suit ce couvercle faisant partie de la soudure de celui-ci au corps principal.

19. Système de pompage volumétrique selon la revendication 9, caractérisé en ce que la face de la partie jetable, face à la pompe, définie par le corps principal et le couvercle supérieur, retenant entre les deux la membrane de distension, est plate, pour permettre à la membrane de se distendre hors de ses limites, son volume d'expansion restant défini par la pompe lorsque cet élément y est compris, pour permettre un contrôle précis dudit volume.

20.     Système de pompage selon la revendication 9, caractérisé en ce que le couvercle arrière a, vers l'extérieur, une entaille centrale définissant une calotte sphérique centrale proéminente, laquelle en guise de loupe permet une plus grande visualisation des bulles à l'intérieur de la cassette durant la purge initiale de celle-ci.

21.     Système de pompage selon la revendication 9, caractérisé en ce que la membrane est circulaire.

22.     Système de pompage selon la revendication 9, caractérisé en ce que les bouchons sont indépendants entre eux et constituent des corps indépendants de la membrane de distension.

0086731

**Fig.1**

Fig. 2

0086731

III/6

33

32

Fig.3

1

11

12

31

33

Fig.3

Fig.4

Fig. 5

Fig.6

0086731